# EUROPEAN PATENT APPLICATION

(11) **EP 4 144 335 A1**
(43) Date of publication of application: **08.03.2023**
(21) Application number: 21797403.9
(22) Date of filing: 19.04.2021
(51) Int. Cl.: A61K 8/02, A61Q 1/10, A61Q 1/12

(54) **METHOD FOR MANUFACTURING SOLID COSMETIC AND PRESS HEAD**

(30) Priority: 30.04.2020 JP 2020080701
(71) Applicant: Shiseido Company, Ltd., Chuo-ku Tokyo 104-0061 (JP)
(72) Inventor: HIROSAKI, Ken, Tokyo 104-0061 (JP); YAMASHITA, Hisayoshi, Tokyo 104-0061 (JP); URASHIMA, Toshifumi, Tokyo 104-0061 (JP); FURUICHI, Yosuke, Tokyo 104-0061 (JP); NISHIYAMA, Yoshihiro, Tokyo 104-0061 (JP)
(74) Representative: Ter Meer Steinmeister & Partner
(86) International application number: PCT/JP2021/015909
(87) International publication number: WO 2021/220869

(57) **Abstract**

In a method of manufacturing a solid cosmetic having a surface decorated with irregularities, the method includes: a pressing step of obtaining a molded body having a surface decorated with irregularities by press-molding a cosmetic composition containing a powder ingredient and an oily ingredient as a binder, using a press head including a press surface having irregularities corresponding to a surface shape of the solid cosmetic and a plurality of through holes penetrating through the press surface; and a mold releasing step of, after the pressing step, separating the press surface and the molded body, wherein the press head is a laminate made by laminating together a plurality of plates with holes, the through holes are formed by connecting the holes of each of the plates, and diameters of the through holes are 50 µm to 300 µm.

## Description

### [Technical Field]

The present invention relates to a method of manufacturing a solid cosmetic and a press head.

### [Background Art]

A solid cosmetic is a cosmetic in a solid state containing powder ingredients, oily ingredients, and the like, and is used in foundations, eye shadows, eye colors, powder eyebrows, and the like. The surface of the solid cosmetic is decorated with decorative patterns such as characters, figures, patterns, and the like, and is given a wide variety of designs.

A solid cosmetic with a decorated surface can be produced, for example, by subjecting the cosmetic filled in a dish-shaped container or the like to a pressing process to be embossed.

As a method of manufacturing a solid cosmetic with a decorated surface, for example, a method of manufacturing a powder solid cosmetic has been proposed in which a porous resin film having pores on the surface is interposed between a powder cosmetic and a compression mold, and the powder cosmetic is compression molded using the mold (see, for example, PTL 1).

### [Citation List]

### [Patent Literature]

[PTL 1]
Japanese Laid-Open Patent Publication No. 2010-215582

### [Summary of Invention]

### [Technical Problem]

However, in the method of manufacturing a powder solid cosmetic disclosed in PTL 1, because the powder cosmetic is pressed by compression molding via the porous resin film on the surface, the corners of irregular shapes such as patterns and characters formed on the surface of the powder cosmetic tend to be rounded due to the porous resin film, which makes it difficult to form a sharp decorative pattern.

Meanwhile, when the powder solid cosmetic is pressed by compression molding without the porous resin film on the surface, because it is difficult to release the compression mold from the surface of the powder cosmetic, the productivity of the solid cosmetic having a decorative pattern on the surface may decrease. In addition, because the powder cosmetic easily adheres to the compression mold, the decorative pattern formed on the surface tends to be chipped, making it difficult to form a sharp decorative pattern.

An object of one aspect of the present invention is to provide a method of manufacturing a solid cosmetic that can produce a solid cosmetic having a sharp decorative processing on the surface with high productivity.

### [Solution to Problem]

One aspect of the present invention is: a method of manufacturing a solid cosmetic having a surface decorated with irregularities, the method including: a pressing step of obtaining a molded body having a surface decorated with irregularities by press-molding a cosmetic composition containing a powder ingredient and an oily ingredient as a binder, using a press head including a press surface having irregularities corresponding to a surface shape of the solid cosmetic and a plurality of through holes penetrating through the press surface; and a mold releasing step of, after the pressing step, separating the press surface and the molded body, wherein the press head is a laminate made by laminating together a plurality of plates with holes, the through holes are formed by connecting the holes of each of the plates, and diameters of the through holes are 50 µm to 300 µm.

### [Advantageous Effects of Invention]

According to one aspect of the present invention, in the method of manufacturing a solid cosmetic, a solid cosmetic having a sharp decorative processing on the surface can be produced with high productivity.

### [Brief Description of the Drawings]

[FIG. 1]

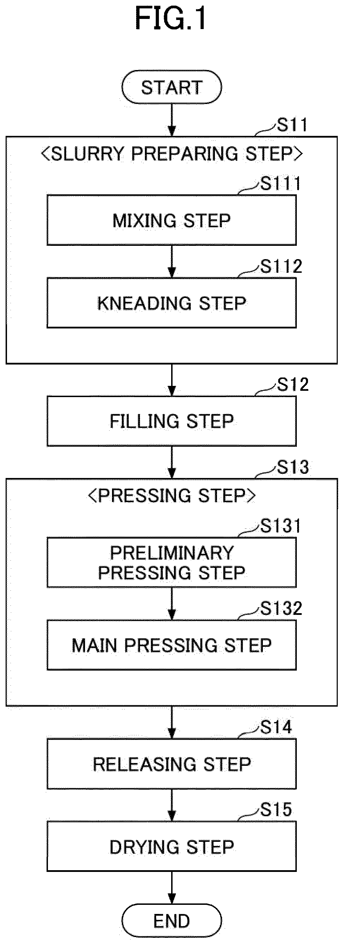

   FIG. 1 is a flowchart illustrating a method of manufacturing a solid cosmetic according to an embodiment of the present invention;
[FIG. 2]

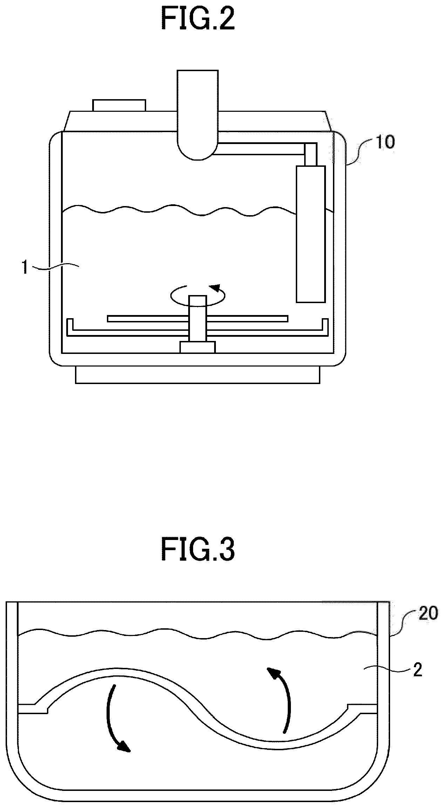

   FIG. 2 is an explanatory diagram illustrating a part of a process;
[FIG. 3]
   FIG. 3 is an explanatory diagram illustrating a part of the process;
[FIG. 4]

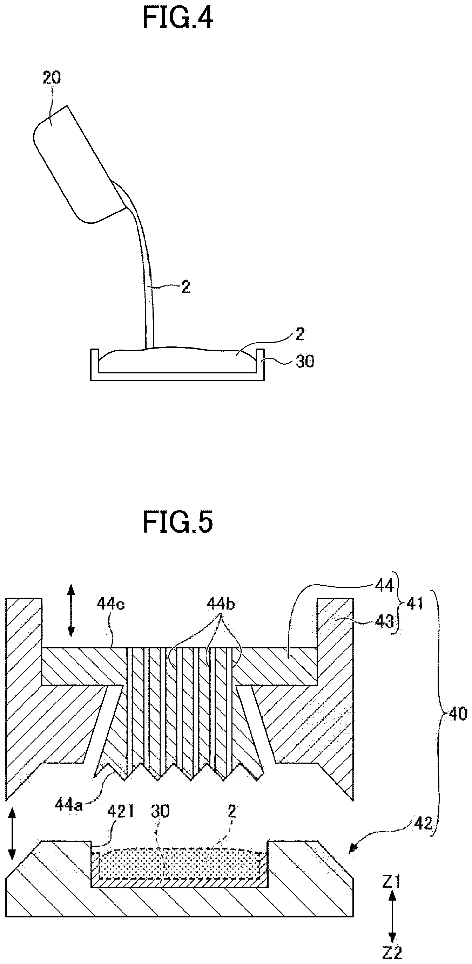

   FIG. 4 is an explanatory diagram illustrating a part of the process;
[FIG. 5]
   FIG. 5 is a diagram illustrating an example of a pressing apparatus;
[FIG. 6]

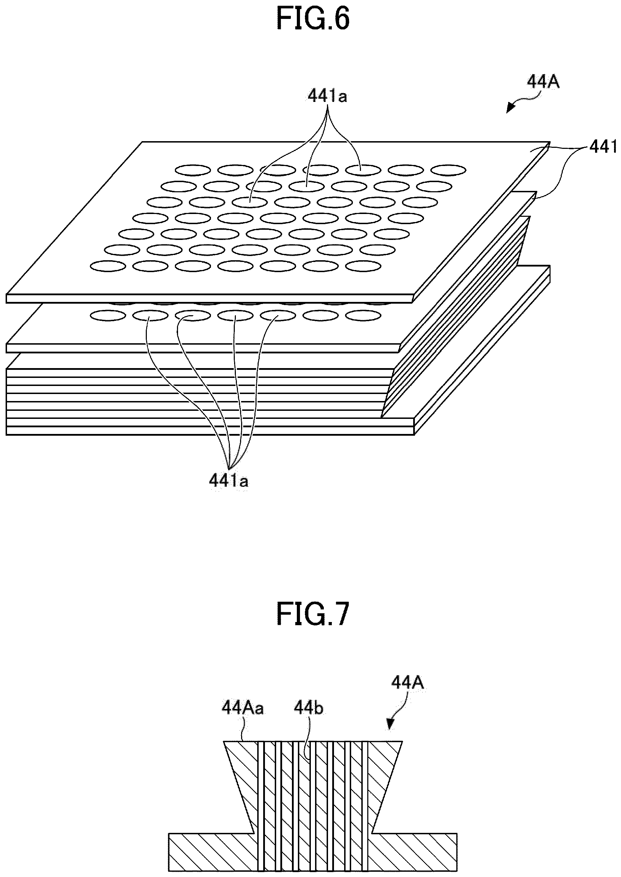

   FIG. 6 is an explanatory diagram illustrating a part of manufacturing a press head;
[FIG. 7]
   FIG. 7 is an explanatory diagram illustrating a part of manufacturing a press head;
[FIG. 8]

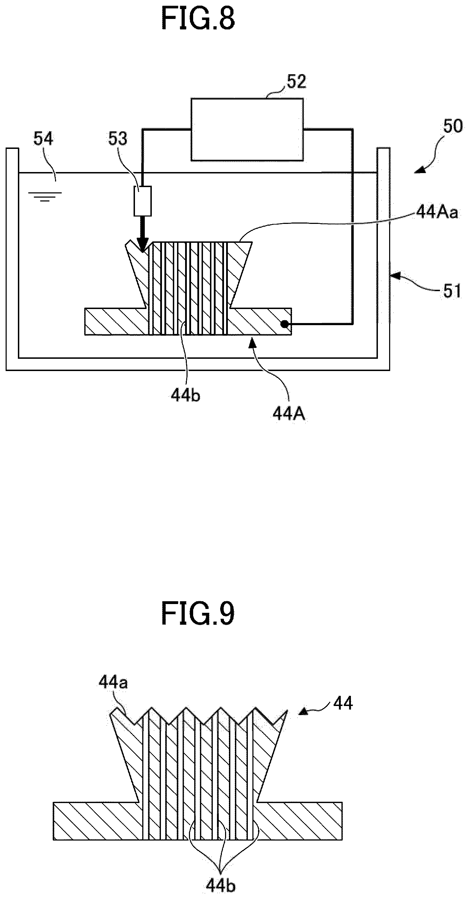

   FIG. 8 is a diagram illustrating an example of an apparatus for forming irregularities on a processing surface of a laminate using an electric discharge machining device;
[FIG. 9]
   FIG. 9 is a diagram illustrating a press head having a press surface formed thereon;
[FIG. 10]

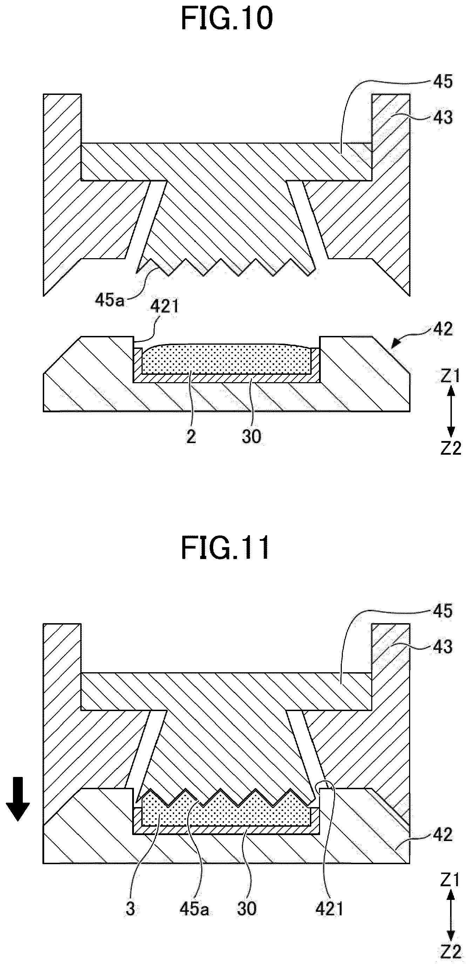

   FIG. 10 is an explanatory diagram illustrating a part of the process;
[FIG. 11]
   FIG. 11 is an explanatory diagram illustrating a part of the process;
[FIG. 12]

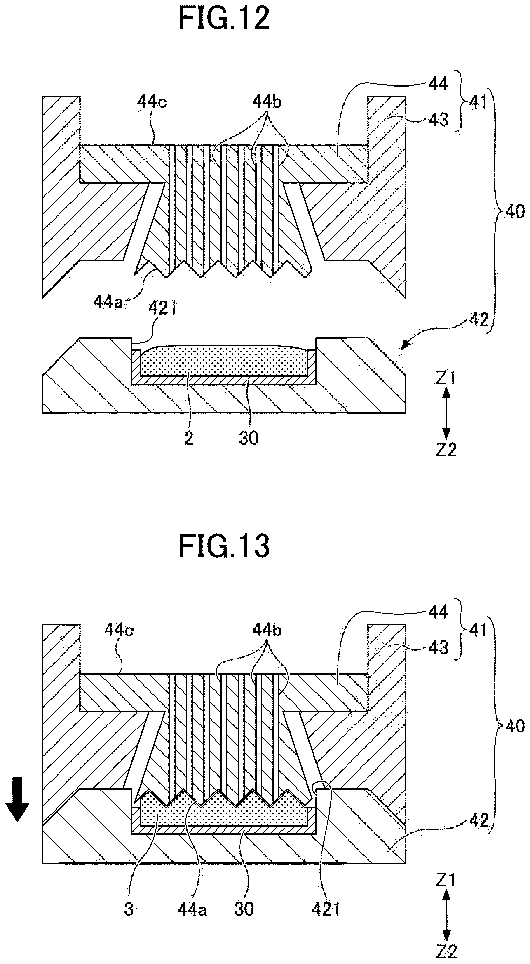

   FIG. 12 is an explanatory diagram illustrating a part of the process;
[FIG. 13]
   FIG. 13 is an explanatory diagram illustrating a part of the process;
[FIG. 14]

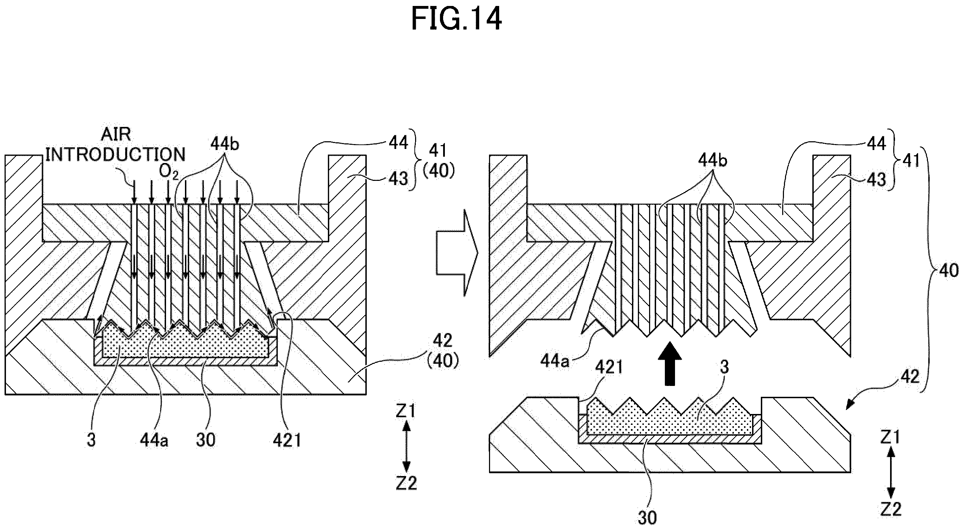

   FIG. 14 is an explanatory diagram illustrating an example in which compressed air is introduced into a through hole toward the press surface;
[FIG. 15]

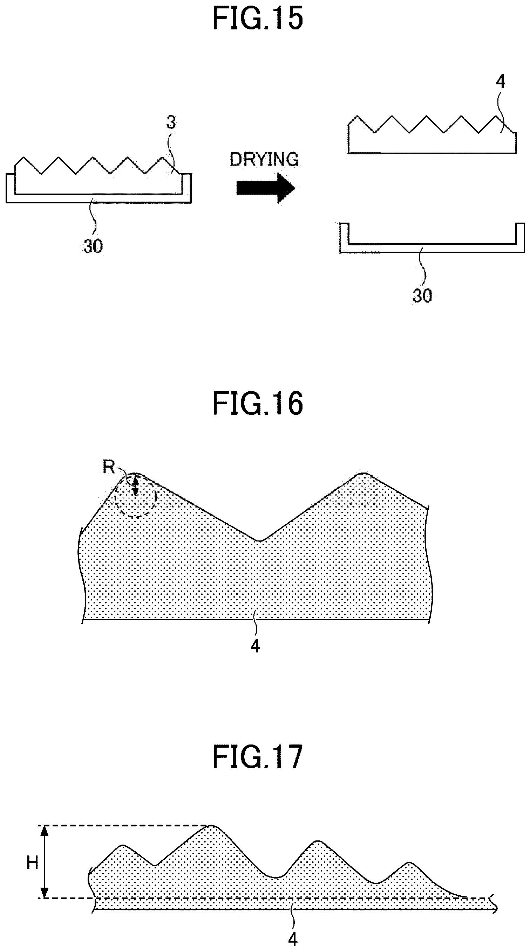

   FIG. 15 is an explanatory diagram illustrating a part of the process;
[FIG. 16]
   FIG. 16 is a diagram illustrating a radius of a corner of a decoration formed on a surface of the solid cosmetic; and
[FIG. 17]
   FIG. 17 is a diagram illustrating a height difference of the decoration formed on the surface of the solid cosmetic.

### [Description of Embodiments]

In the following, embodiments of the present invention will be described in detail. In order to facilitate understanding of the description, the same components are denoted by the same reference numerals in each drawing, and overlapping descriptions are omitted. The scale of each member in the drawings may differ from the actual scale. As used herein, "X to Y" indicating a numerical range is meant to include a lower limit and an upper limit that are given as numerical values before and after "to", unless otherwise noted.

### <Method of Manufacturing Solid Cosmetic>

A method of manufacturing a solid cosmetic according to an embodiment of the present invention will be described.

The solid cosmetic is a cosmetic composition (mixture) containing powder ingredients, oily ingredients, optional ingredients, if necessary, and the like, and formed into a solid. Examples of the solid cosmetic include makeup cosmetics such as foundation, white powder, eye shadow, eye color, eyeliner, cheek color, eyebrow, powder, blush, lipstick, and the like, and basic cosmetics such as body powder, whitening powder, deodorant powder, fragrance powder, and the like. In the present embodiment, an example will be described in which the powder cosmetic is an eye shadow.

As used herein, a cosmetic composition includes powder ingredients, oily ingredients, optional ingredients, and the like. The form of the cosmetic composition is not limited. The cosmetic composition includes: a cosmetic mixture powder mixed with powder ingredients, oily ingredients, optional ingredients, and the like; a slurry in which the cosmetic mixture powder is mixed and dispersed in a volatile dispersion medium; and a semi-dry product (molded body) formed by drying at least part of the volatile dispersion medium contained in the slurry.

In the method of manufacturing a solid cosmetic according to the present embodiment, a wet process can be used in which a slurry, capillary, or the like are obtained by adding a suitable amount of the cosmetic composition to a solvent and mixing them, and then the solvent is removed, or, a dry process can be used in which the cosmetic composition is filled into a container and compressed. When the wet process is used, a slurry, capillary, or the like may be used depending on the amount of the solvent to which the cosmetic composition is added. In the present embodiment, a case where the wet process is used will be described.

FIG. 1 is a flowchart illustrating a method of manufacturing a solid cosmetic according to an embodiment of the present invention. As illustrated in FIG. 1, the method of manufacturing a solid cosmetic according to the present embodiment is a method of manufacturing a solid cosmetic having a surface decorated with irregularities, and includes a slurry preparing step (Step S11), a filling step (Step S12), a pressing step (Step S13), a mold releasing step (Step S14), and a drying step (Step S15) .

Hereinafter, each process will be described with reference to FIGS. 2 to 15. FIGS. 2 to 15 are explanatory diagrams of each step of the method of manufacturing a solid cosmetic according to the embodiment of the present invention.

The slurry preparing step (Step S11) is a step of obtaining a slurry containing powder ingredients, and includes a mixing step (Step Sill) and a kneading step (Step S112), as illustrated in FIG. 1.

In the mixing step (Step S111), as illustrated in FIG. 2, a cosmetic mixture powder 1 is obtained by filling a mixer 10 with a plurality of types of powder ingredients, an oily ingredient, and optional ingredients, if necessary, and mixing them. When the oily ingredient contains a solid or paste oil at room temperature (25°C ± 2°C), it is preferable that the oily ingredient is heated and melted, and then dry mixed with the powder ingredient. As the optional ingredient, for example, a thickening agent may be contained. Accordingly, the bonding between the powder ingredients becomes stronger, and the hardness (viscosity) can be adjusted to be appropriate for molding the cosmetic mixture powder 1.

As the mixer 10, a known mixer, such as Henschel mixer (registered trade mark) or a pulverizer may be used.

The mixing ratio of the powder ingredients to the oily ingredient is appropriately selected according to the type of the powder ingredients and the oily ingredient to be used. For example, it is preferable that the ratio of the powder ingredients to the oily ingredient is between 60:40 to 99.5:0.5 by mass.

In the kneading step (Step S112), as illustrated in FIG. 3, the cosmetic mixture powder 1 produced in the mixing step (Step Sill) and the volatile dispersion medium are filled into a kneading machine 20, and the cosmetic mixture powder 1 is dispersed in the volatile dispersion medium to produce a slurry 2. The slurry 2 obtained is moved to a storage tank or the like for storage.

As the volatile dispersion medium, a volatile organic solvent, water, and the like may be used. Examples of the volatile organic solvent include low boiling point alcohols such as ethyl alcohol, acetone, isopropyl alcohol, n-butanol; low boiling point hydrocarbon oils such as xylene, toluene, benzene, heptane, pentane, hexane, cyclohexane, isooctane, nonane, decane, p-menthane, pinene, isododecane, isohexadecane, isoparaffin, limonene; low boiling point, chained or cyclic silicone oils such as dimethyl polysiloxane, methyl trimethicone, hexamethyl cyclotrisiloxane, octamethyl cyclotetrasiloxane, decamethyl cyclopentasiloxane; low boiling point fluorine compounds such as perfluoropolyether, and the like. These solvents may be used alone or in combination with two or more.

The content of the volatile dispersion medium may be appropriately selected depending on the polarity and specific gravity of the volatile dispersion medium to be used, as long as sufficient fluidity of the slurry 2 is ensured when the slurry 2 is filled into an inner plate 30 in the filling step (Step S12), described later. The content of the volatile dispersion medium is preferably, for example, about half to twice of the cosmetic mixture powder 1 in the mass ratio.

As the kneading machine 20, a known kneading machine, such as a kneader, a biaxial kneading machine, a dispersion mixer, a homogenizer, a planetary mixer, COMBI MIX (registered trade mark), AGI-HOMO mixer, and the like may be used.

In the filling step (Step S12), as illustrated in FIG. 4, the slurry 2 produced in the kneading step (Step S112) is filled into the inner plate 30 made of metal or resin.

In the pressing step (Step S13), the slurry 2 filled in the inner plate 30 is pressed and molded using a pressing apparatus to obtain a molded body 3 (see FIG. 13, and the like), which is a semi-dry product having a surface decorated with irregularities.

### (Pressing Apparatus)

The configuration of the pressing apparatus will now be described.

FIG. 5 is a diagram illustrating an example of a pressing apparatus. In FIG. 5, the dashed line indicates the inner plate 30 and the slurry 2 filled into the inner plate 30. As illustrated in FIG. 5, the pressing apparatus 40 includes an upper pressing apparatus 41 and a lower pressing apparatus 42. During pressing, the upper pressing apparatus 41 is arranged above (upper side in FIG. 5) the lower pressing apparatus 42.

The upper pressing apparatus 41 includes a body 43 and a press die (press head) 44, as illustrated in FIG. 5.

The press head 44 is configured to move in a vertical direction (Z1 and Z2 direction) with respect to the body 43.

The press head 44 includes, as illustrated in FIG. 5, a press surface 44a having a decorative pattern (irregularities) to be transferred to the slurry 2 and a plurality of through holes 44b penetrating through the press surface 44a.

The irregularities formed on the press surface 44a is designed according to the shape of the decoration formed on the solid cosmetic.

The through holes 44b open at one end to the press surface 44a and at the other end to the back surface 44c of the press head 44. The through holes 44b are arranged to open at a substantially uniform density on the press surface 44a.

The pore size of the through hole 44b is 50 µm to 300 um, preferably 80 um to 200 µm, and more preferably 100 um to 150 um. When the pore size of the through hole 44b is 50 um to 300 um, it is possible to reduce the possibility that traces of the through holes 44b remain on the slurry 2 due to the slurry 2 entering the through-holes 44b.

The ratio of the interval between the through holes 44b to the size of the through holes 44b is preferably 4:1 to 2:3. The interval between the through holes 44b refers to the center interval between the adjacent through holes 44b. When the ratio of the interval between the through holes 44b and the size of the through holes 44b is within the preferred range described above, the strength of the press head 44 is prevented from being lowered, and the durability of the press head 44 can be maintained. Further, when the ratio of the interval between the through holes 44b and the size of the through holes 44b is within the preferred range described above, when the compressed air is supplied from the through holes 44b, it becomes easy to supply the necessary amount of air to separate the contact surface between the press surface 44a and the semi-dry molded body 3 (see FIG. 13 and the like). The interval between the through holes 44b varies depending on the size of the through holes 44b, but is preferably as narrow as possible. For example, when the size of the through holes 44b is 100 µm, the interval between the adjacent through holes 44b is preferably 200 µm to 250 µm.

As illustrated in FIG. 5, the lower pressing apparatus 42 includes a mounting recess 421 formed at a position facing the press surface 44a when the upper pressing apparatus 41 is mounted. In the mounting recess 421, the inner plate 30 filled with the slurry 2 is mounted. Therefore, the press head 44 is configured to move in the vertical direction (in the Z1 and Z2 directions) with respect to the mounting recess 421.

The configuration of the press head 44 and a method of manufacturing the same will be described.

The press head 44 is formed from a laminate 44A made by laminating together a number of plates 441 with holes as illustrated in FIG. 6. By forming the press head 44 from the laminate 44A of a number of plates 441 with holes, as illustrated in FIG. 5, even when the press head 44 is formed thick, the press head 44 is ensured to have a number of through holes 44b at small intervals.

The plate 441 with holes is, for example, a conductive metal plate with a thickness of 0.1 mm, and is formed with a number of pores (hereinafter, simply referred to as holes) 441a, as illustrated in FIG. 6. The diameter of the hole 441a corresponds to the pore size of the through hole 44b, and is 50 um to 300 µm, preferably 80 um to 200 µm, and more preferably 100 um to 150 µm.

The holes 441a formed in the plate 441 with holes are formed so that the positions of the holes 441a formed in each of the plates 441 correspond to each other when the plates 441 are laminated. Accordingly, through holes 44b (see FIG. 5) are formed by laminating each of the plates 441 with holes and connecting the holes 441a of each of the plates 441.

The method of bonding the plurality of plates 441 with holes may be any method as long as the plurality of plates 441 with holes can be bonded, for example, by diffusion bonding. Specifically, a plurality of positioned and laminated plates 441 with holes are placed in a vacuum atmosphere or an inert gas atmosphere, and the plates 441 are laminated while heating at a temperature below the melting point of the material forming the plates 441 and pressurizing the laminate 44A of the plates 441 at a pressure that does not cause plastic deformation. As a result, diffusion of atoms occurs on the bonding surfaces of the respective plate 441, and the opposed plates 441 are stuck together by diffusion bonding. The laminate 44A is formed by laminating together a plurality of plates 441 by diffusion bonding.

Next, as illustrated in FIG. 7, irregularities (patterns) for decorating the slurry 2 are formed on the processing surface 44Aa of the laminate 44A (the surface to be the press surface 44a of the press head 44). At this time, the through holes 44b are opened in the processing surface 44Aa. Therefore, when forming the irregularities on the processing surface 44Aa for decorating, it is necessary to form the irregularities so as not to collapse the openings of the through holes 44b in the processing surface 44Aa.

As a method of forming the irregularities on the processing surface 44Aa, electric discharge machining, cutting machining, press machining, laser machining, and the like may be used. Among these, electric discharge machining is preferably used. In a method such as cutting machining, press machining, or the like, in which a cutting jig or a press jig is directly brought into contact with the processing surface 44Aa, or in a method in which the processing surface 44Aa is melted, such as laser processing, the openings of the through holes 44b in the processing surface 44Aa may be collapsed. When electric discharge machining is used, irregularities (patterns) can be formed on the processing surface 44Aa without collapsing the openings of the through holes 44b on the processing surface 44Aa. Therefore, it is preferable to use electric discharge machining.

An example of a method of forming irregularities (patterns) on the processing surface 44Aa using electric discharge machining will be described.

FIG. 8 is a diagram illustrating an example of an apparatus for forming irregularities on the processing surface 44Aa of the laminate 44A using an electric discharge processing device 50. As illustrated in FIG. 8, the electric discharge processing device 50 includes a container 51, a power supply 52, an electrode 53, and the like.

The container 51 is a container for filling a discharge liquid 54 which is a dielectric. The laminate 44A is immersed in the discharge liquid 54 in the container 51.

The power supply 52 is a power supply for performing discharge machining, one electrode (for example, a negative electrode) being connected to the laminate 44A and the other end (for example, a positive electrode) being connected to the electrode 53.

The electrodes 53 are arranged so as to be close to the processing surface 44Aa.

When the power supply 52 is driven, arc discharge is generated between the electrode 53 and the processing surface 44Aa, and part of the processing surface 44Aa melts and evaporates. As a result, fine irregularities are formed on the processing surface 44Aa. Particles evaporated on the surface of the processing surface 44Aa are washed away by the discharge liquid 54 and do not remain on the processing surface 44Aa.

The electrode 53 is configured to move above the processing surface 44Aa by a moving device (not illustrated). The moving device is connected to a control device (not illustrated) in which pattern data to be formed on the processing surface 44Aa is stored. By controlling the moving device of the electrode 53 and the electric discharge processing device 50, the processing surface 44Aa of the laminate 44A is automatically formed with irregularities corresponding to the pattern data.

**FIG.** 9 is a diagram illustrating the press head 44 having the press surface 44a formed thereon. As illustrated in FIG. 9, when using electric discharge machining, the press head 44 having the press surface 44a with a decorative pattern (irregularities) is manufactured. On the press surface 44a, irregularities corresponding to the decorative pattern can be formed without collapsing the through holes 44b. Accordingly, by using electric discharge machining to form the press surface 44a, it is possible to form the press surface 44a having irregularities corresponding to the decorative pattern without collapsing the through holes 44b.

Next, the pressing step (Step S13) includes a preliminary pressing step (Step S131) and a main pressing step (Step S132).

In the preliminary pressing step (Step S131), the slurry 2 is preliminarily pressed using a preliminary press head. The slurry 2 is temporarily pressed while being semi-dried, and a semi-dry product of the slurry 2 is obtained. In the preliminary pressing step (Step S131), by temporarily pressing the slurry 2 while the slurry 2 is semi-dried, when the main pressing is performed using the press head 44 in the main pressing step (Step S132), it is possible to reduce the adhesion of the slurry 2 to the press surface 44a of the press head 44.

In the preliminary pressing step (Step S131), as illustrated in FIG. 10, the inner plate 30 filled with slurry 2 is mounted in the mounting recess 421. The preliminary press head 45 and the lower pressing apparatus 42 are arranged so that the press surface 45a of the preliminary press head 45 faces the inner plate 30 on the mounting recess 421. The preliminary press head 45 is the same as the press head 44 constituting the upper pressing apparatus 41 except that through holes are not provided. Thus, the configuration of the preliminary press head 45 and the manufacturing method thereof will not be described.

Then, as illustrated in FIG. 11, the preliminary press head 45 is moved toward the inner plate 30 (in the Z2 direction), and the press surface 45a is brought into contact with the slurry 2 filled in the inner plate 30 to preliminarily press the slurry 2. Thus, the semi-dry product in which the slurry 2 is semi-dried can be obtained.

The main pressing step (Step S132) is performed using the pressing apparatus 40 illustrated in FIG. 5, and the semi-dry product of the slurry 2 obtained in the preliminary pressing step (Step S131) is pressed.

In the main pressing step (Step S132), first, as illustrated in FIG. 12, the inner plate 30 filled with the slurry 2 is mounted in the mounting recess 421. The upper pressing apparatus 41 and the lower pressing apparatus 42 are arranged so that the press surface 44a of the upper pressing apparatus 41 faces the inner plate 30 on the mounting recess 421.

Then, as illustrated in FIG. 13, the press head 44 is moved toward the inner plate 30 (in the Z2 direction), the press surface 44a is brought into contact with the slurry 2 filled in the inner plate 30, and the slurry 2 is pressed while being semi-dried. By pressing the slurry 2 with the press surface 44a while the slurry 2 is semi-dried, the molded body 3, which is a semi-dry product of the slurry 2, can be pressed to have a shape corresponding to the press surface 44a. As a result, the decorative pattern (irregularities) to be transferred formed on the press surface 44a can be transferred to the molded body 3 in a semi-dry state from which at least part of the volatile dispersion medium has been removed. Accordingly, the molded body 3 having a surface decorated with irregularities is obtained.

In the main pressing step (Step S132), when the slurry 2 is pressed by the press head 44, there is a possibility that air remains between the slurry 2 and the press surface 44a. Because the press head 44 has through holes 44b on the press surface 44a, when the slurry 2 is pressed by the press head 44 in the main pressing step (Step S132), the air present between the slurry 2 and the press surface 44a can escape to the outside through the through holes 44b. Therefore, because the residual air between the slurry 2 and the press surface 44a can be drawn out, the irregularities of the press surface 44a can be transferred to the molded body 3 with high accuracy. Further, it is possible to reliably release the press surface 44a of the press head 44 from the molded body 3 without interposing a release sheet (also referred to as wrapping paper) between the press surface 44a and the molded body 3.

Further, it is preferable that the through holes 44b are arranged so as to open at a substantially uniform density on the press surface 44a. In this case, the residual air between the slurry 2 and the press surface 44a can be uniformly drawn out on the entire surface of the joint position between the press surface 44a and the molded body 3. Accordingly, it is possible to reduce uneven transfer of the irregularities of the press surface 44a to the molded body 3 due to residual air between the slurry 2 and the press surface 44a. The decoration formed on the molded body 3 does not collapse due to non-uniform application of force for separating the press surface 44a from the molded body 3.

The hardness of the molded body 3 can be adjusted by adjusting the amount of the thickener, which is an optional ingredient, the processing rotation speed of the kneading machine 20, the amount of the volatile dispersion medium, and the like.

In the mold releasing step (Step S14), as illustrated in FIG. 14, a mold releasing processing is performed in which the press head 44 is separated from the molded body 3. By moving the press head 44 upward and separating the upper pressing apparatus 41 from the lower pressing apparatus 42 to separate the press surface 44a from the molded body 3, the molded body 3 having a decorated surface is formed.

When releasing the press head 44 from the molded body 3, compressed air may be introduced into the through holes 44b formed in the press head 44. Introduction of compressed air into the through holes 44b may be performed, for example, using a compressed air generator such as a compressor and the like. The compressed air introduced into the through holes 44b can act as a force to separate the press surface 44a and the molded body 3 at the interface between the press surface 44a and the molded body 3. Therefore, it is possible to reliably release the press surface 44a of the press head 44 from the molded body 3 without interposing a release sheet (also referred to as wrapping paper) between the press surface 44a and the molded body 3.

In addition, the air pressure of the compressed air may be set at a pressure in a range in which the decorative pattern formed on the surface of the molded body 3 is not deformed. Even when compressed air is injected onto the surface of the molded body 3 on which the decoration processing has been performed when releasing, the molded body 3 has a predetermined viscosity, so that the molded body 3 can maintain its form to some extent. Therefore, even when the press head 44 is separated from the molded body 3 using compressed air, the compressed air does not damage the decorated surface of the molded body 3.

In the drying step (Step S15), as illustrated in FIG. 15, the molded body 3 is dried at a predetermined drying temperature and drying time. As a result, a solid cosmetic 4 having a surface decorated with irregularities is obtained.

The drying temperature and drying time may be appropriately selected depending on the content of the powder ingredient, the oily ingredient, and the optional ingredient, which constitute the molded body 3, the shape and size of the molded body 3, and the like. The drying temperature of the molded body 3 is preferably, for example, drying at 20°C to 80°C. The drying time depends on the type of aqueous solvent and the drying temperature, but is preferably between 5 hours and 30 hours, for example. Blotting paper may be placed on the surface prior to drying, with application of pressure to remove some of the solvent.

As described above, the method of manufacturing a solid cosmetic according to the present embodiment includes a pressing step (Step S13) and a mold releasing step (Step S14). In the pressing step (Step S13), using the press head 44, the surface of the molded body 3, obtained from the slurry 2 being semi-dried, is decorated with irregularities. Then, in the mold releasing step (Step S14), the press surface 44a of the press head 44 and the molded body 3 are separated from each other to obtain the solid cosmetic 4 to which decoration is applied. The press head 44 is composed of a laminate 44A formed by laminating a plurality of plates 441 with holes. The diameter of the through holes 44b formed by connecting the holes 441a of the plates 441 with holes is 50 µm to 300 µm.

According to the method of manufacturing a solid cosmetic according to the present embodiment, the diameter of the through hole 44b is 50 um to 300 µm, so that when the slurry 2 is pressed, air present between the slurry 2 and the press surface 44a can easily pass out through the through hole 44b together with the volatile dispersion medium, thereby reducing the residual air between the slurry 2 and the press surface 44a. Therefore, it is possible to accurately transfer the irregularities formed on the press surface 44a to the molded body 3. When the molded body 3 is separated from the press surface 44a, the press surface 44a can be separated from the molded body 3 without breaking the decoration formed on the surface of the molded body 3. Therefore, as illustrated in FIG. 16, because the radius R of the corner of the decoration formed on the surface of the solid cosmetic 4 can be reduced, the angle of the raised corner of the convex portion formed on the surface of the solid cosmetic 4 can be sharpened. Therefore, according to the method of manufacturing the solid cosmetic according to the present embodiment, the solid cosmetic 4 having the sharp decorative processing on the surface can be formed with high productivity.

Further, according to the method of manufacturing a solid cosmetic according to the present embodiment, the maximum height difference of the irregularities of the decoration formed on the surface of the solid cosmetic 4 can be increased to, for example, 3 mm or more. The maximum height difference of the irregularities of the decoration is preferably 5 mm or more, and more preferably 10 mm or more. As illustrated in FIG. 17, when the maximum height difference H of the irregularities of the decoration formed on the surface of the solid cosmetic 4 is increased to, for example, 3 mm or more, the decoration with a larger height difference can be formed.

When the method of manufacturing a solid cosmetic according to the present embodiment is used, the obtained solid cosmetic 4 can have the impact resistance required for use. Accordingly, according to the method of manufacturing a solid cosmetic according to the present embodiment, the solid cosmetic 4 has a small radius of corners, has the decoration with a large height difference of the irregularities, and has a strength that satisfies the standard for use.

According to the method of manufacturing a solid cosmetic according to the present embodiment, the solid cosmetic 4 may include, on the surface, an area where a radius R (see FIG. 16) of a corner of the decoration is 0.2 mm or less. Accordingly, in the decoration formed on the surface of the solid cosmetic 4, the angle of the raised corner of the convex portion can be made sharper, and the decoration with sharper corners can be formed.

According to the method of manufacturing a solid cosmetic according to the present embodiment, the surface of the slurry 2 may be directly contacted with the press surface 44a to press the slurry 2. Therefore, in the method of manufacturing a solid cosmetic according to the present embodiment, the surface of the molded body 3 can be pressed and decorated without using a release sheet. When the slurry 2 is pressed, in a case where a release sheet is arranged between the molded body 3 and the press surface 44a, and the press surface 44a is pressed against the surface of the molded body 3, releasing is relatively easy because the molded body 3 and the press surface 44a do not come into direct contact with each other. However, even when the press surface 44a is pressed against the molded body 3 with the release sheet interposed between the molded body 3 and the press surface 44a, the release sheet is difficult to deform along the irregularities, and the corners of the irregularities transferred to the molded body 3 are likely to be rounded. Therefore, the molded body 3 does not have a sharp edge portion, and a sharp decorative pattern cannot be formed. In contrast, in the present embodiment, the surface 44a of the press head 44 is directly in contact with the slurry 2 while the slurry 2 is semi-dried without using the release sheet, and the decorative pattern of the press surface 44a is transferred to the molded body 3. Therefore, it is possible to sufficiently transfer the sharp shape of the edge portion to the surface of the resulting solid cosmetic 4, and chipping can be reduced. In addition, it is possible to prevent paper scratches caused by deflection of a release sheet from being formed on the surface of the solid cosmetic 4, when the slurry 2 is pressed while the slurry 2 is semi-dried with the release sheet being interposed between the slurry 2 and the press surface 44a.

In the method of manufacturing a solid cosmetic according to the present embodiment, the pressing step (Step S13) may include the preliminary pressing step (Step S131) and the main pressing step (Step S132). In the preliminary pressing step (Step S131), the slurry 2 is made semi-dry, and in the main pressing step (Step S132), main pressing is performed on the semi-dry product of the slurry 2 using the press head 44. Accordingly, it is possible to reduce the adhesion of the slurry 2 to the press surface 44a of the press head 44.

In the method of manufacturing a solid cosmetic according to the present embodiment, the press head 44 in which the interval between the adjacent through holes 44b is 200 µm to 250 µm on the press surface 44a may be used. Accordingly, the contact surface of the press surface 44a and the molded body 3 can be substantially uniformly separated by compressed air supplied from the through holes 44b. In addition, the air present between the press surface 44a and the molded body 3 and the volatile dispersion medium included in the slurry 2 can be drawn through the through holes 44b substantially uniformly from the contact surface between the press surface 44a and the slurry 2.

In the method of manufacturing a solid cosmetic according to the present embodiment, in the mold releasing step (Step S14), releasing may be performed by introducing air from the through holes 44b. Accordingly, because the press head 44 can be released from the molded body 3 without breaking the corners of the molded body 3, a decoration having a sharp angle can be stably formed on the solid cosmetic 4.

Because the method of manufacturing a solid cosmetic according to the present embodiment has the characteristics as described above, it can be effectively used for a solid powder to which a surface is decorated, in addition to the solid cosmetic.

In the present embodiment, the cosmetic composition is not required to be used as a slurry. For example, the cosmetic composition may be used as a clay substantially corresponding to the state of the molded body 3 in which the slurry 2 is semi-dried.

The present embodiment is not limited to the wet process. A dry process for filling and compressing the cosmetic composition into a container may be used.

### <Solid Cosmetic>

A solid cosmetic obtained by using the method of manufacturing a solid cosmetic according to the present embodiment will be described. The solid cosmetic may contain powder ingredients, oily ingredients, and optional ingredients. The ingredients contained in the solid cosmetic will be explained.

### (Powder Ingredients)

The powder ingredients are not particularly limited as long as they are commonly used. Examples of the powder ingredients include talc, kaolin, sericite, muscovite, phlogopite, synthetic mica, synthetic gold mica, synthetic fluorine mica, lepidolite, biotite, calcined talc, calcined sericite, calcined muscovite, calcined phlogopite, vermiculite, magnesium carbonate, calcium carbonate, aluminum silicate, barium silicate, calcium silicate, magnesium silicate, strontium silicate, tungstate, magnesium, silica, zeolite, barium sulfate, calcined calcium sulfate, calcium phosphate, fluorine apatite, hydroxyapatite, ceramic powder, lime soaps (for example, calcium myristate, zinc myristate, calcium palmitate, aluminum stearate, and the like), boron nitride, photochromic titanium oxide (titanium dioxide sintered with iron oxide), reduced zinc oxide; organic powder (for example, silicone elastomer powder, silicone powder, silicone resincoated silicone elastomer powder, polyamide resin powder (nylon powder), polyethylene powder, polystyrene powder, copolymer resin powder of styrene and acrylic acid, benzoguanamine resin powder, polytetrafluoroethylene powder, cellulose powder, and the like); inorganic white pigments (for example, titanium dioxide, zinc oxide, and the like); inorganic red pigments (for example, iron oxide (colcothar), iron titanate, and the like); inorganic brown pigments (for example, γ-iron oxide, and the like); inorganic yellow pigments (for example, yellow iron oxide, ocher, and the like); inorganic black pigments (for example, black iron oxide, low valence titanium oxide, and the like); inorganic purple pigments (for example, mango violet, cobalt violet, and the like); inorganic green pigments (for example, chromium oxide, chromium hydroxide, cobalt titanate, and the like); inorganic blue pigments (for example, ultramarine, iron blue, and the like); pearl pigments (for example, bismuth oxychloride, argentine, mica titanium, iron oxide coated mica titanium, low valence titanium oxide coated mica titanium, photochromic mica titanium, compounds that use talc, glass, synthetic fluorine phlogopite, silica, bismuth oxychloride, and the like, instead of mica as substrate, compounds that use low valence titanium oxide, colored titanium oxide, iron oxide, alumina, silica, zirconia, zinc oxide, cobalt oxide, aluminum, and the like, instead of titanium oxide as the coating, functional pearl pigments such as compounds in which resin particles are coated on the surface of the pearl pigment, aluminum hydroxide particles are coated on the surface of the pearl pigment, zinc oxide particles are coated on the surface of the pearl pigment, barium sulfate particles are coated on the surface of the pearl pigment, and the like; metal powder pigments (for example, aluminum powder, copper powder, and the like); organic pigments such as zirconium, barium, or aluminum lake (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow 401, and Blue 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, and the like); natural pigments (for example, chlorophyll, β-carotene, and the like), and the like.

In the present embodiment, it is preferable to contain a hydrophobic powder and/or a hydrophobized powder, and these may be contained in large amounts. The hydrophobic powder or the hydrophobized powder used in the present embodiment refers to the powder having a low affinity for water. The hydrophobic powder is a powder having a low affinity for water, and the hydrophobized powder is a powder that is made hydrophobic by surface-treating a powder that has a high affinity for water. The hydrophobicity is determined by the following method. That is, 50 g of ion-exchange water and 0.1 g of the powder to be evaluated are stored in a transparent, sealed container, and stored at 50°C for 1 day, followed by visual observation. When the majority of the powder to be evaluated is present on the surface of the ion-exchange water, it is evaluated to be hydrophobic.

Examples of the hydrophobizing treatment of the powder include surface treatment of the powder with a higher fatty acid, a metal stone, an oil, a wax, a silicone compound, a fluorine compound, a hydrocarbon, a surfactant, a dextrin fatty acid ester, and the like. Among these, silicone compound treatment is preferable. In particular, it is preferable that a powder surface-treated with carboxy silicone soap (a metal salt of the terminal carboxyl group of the carboxy-modified silicone) is highly blended (as a guideline, for example, 15% by mass to 25*%* by mass of the total amount of the solid powder cosmetic), because the strength of the solid powder cosmetic is improved.

### (Oily Ingredients)

The oily ingredients are not particularly limited as long as they are commonly used. Examples of the oily ingredients include liquid fats and oils, solid fats and oils, wax, hydrocarbons, higher fatty acids, higher alcohols, synthetic ester oils, silicone oils, and the like. As used herein, oils and oil-soluble ingredients are also referred to as oily ingredients.

Examples of the liquid fats and oils include avocado oil, camellia oil, turtle oil, macadamia nut oil, corn oil, mink oil, olive oil, rapeseed oil, egg yolk oil, sesame oil, persic oil, wheat germ oil, camellia kissi seed oil, castor oil, linseed oil, safflower oil, cottonseed oil, perilla oil, soybean oil, peanut oil, tea seed oil, kaya oil, rice bran oil, palm kernel oil, tung oil, jojoba oil, germ oil, triglycerol, and the like. Examples of the solid fats and oils include cocoa butter, palm oil, horse tallow, hardened palm oil, palm oil, beef tallow, lamb tallow, hardened beef tallow, palm kernel oil, pork fat, beef bone fat, Japanese wax tree kernel oil, hardened oil, beef leg sesame, Japan wax, hardened castor oil, and the like.

Examples of the waxes include beeswax, candelilla, cotton wax, carnauba wax, bayberry wax, insect wax, spermaceti, montan wax, bran wax, lanolin, kapok wax, lanolin acetate, liquid lanolin, sugar cane wax, lanolin fatty acid isopropyl ester, hexyl laurate, reduced lanolin, jojoba wax, hardened lanolin, shellac wax, polyoxyethylene (hereinafter referred to as POE) lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils include liquid paraffin, ozocerite, squalane, pristane, paraffin, ceresin, squalene, vaseline, microcrystalline wax, and the like.

Examples of the higher fatty acids include lauric acid, myristic acid, palmitic acid, stearic acid, behenic acid, oleic acid, undecylenic acid, tall oil acid, isostearic acid, linoleic acid, linolenic acid, eicosapentaenoic acid (EPA), docosahexaenoic acid (DHA), and the like.

Examples of the higher alcohols include straight chain alcohols (for example, lauryl alcohol, cetyl alcohol, stearyl alcohol, behenyl alcohol, myristyl alcohol, oleyl alcohol, cetostearyl alcohol, and the like); branched chain alcohols (for example, glyceryl monostearyl ether (bacyl alcohol), 2-decyltetradecinol, lanoline alcohol, cholesterol, phytosterol, hexyldodecanol, isostearyl alcohol, octyldodecanol, and the like).

Examples of the synthetic ester oils include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, di-2-ethylhexanoic acid ethylene glycol, dipentaerythritol fatty acid ester, N-alkylglycol monoisostearate, neopentyl glycol dicaprate, diisostearyl malate, glyceryl di-2-heptylundecanoate, trimethylol propane tri-2-ethyl hexanoate, trimethylol propane triisostearate, pentaerythritol tetra(2-ethylhexanoate), glyceryl tri(2-ethylhexanoate), glyceryl trioctanoate, glyceryl triisopalmitate, trimethylolpropane triisostearate, cetyl 2-ethylhexanoate, 2-ethylhexylpalmitate, glyceryl trimyristate, tri-2-heptylundecanoate, castic oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyllaurate, di-2-ethylhexyl sebacate, 2-hexyldecyl myristate, 2-hexyldecyl palmitate, 2-hexyldecyl adipatate, diisopropyl sebacate, 2-ethylhexyl succinate, triethyl citrate, and the like.

Examples of the silicone oil include dimethyl polysiloxane, methyl hydrogen polysiloxane, methylphenyl polysiloxane, stearoxymethyl polysiloxane, polyether modified organopolysiloxane, fluoroalkyl polyoxyalkylene co-modified organopolysiloxane, alkyl modified organopolysiloxane, terminal unmodified organopolysiloxane, fluorine modified organopolysiloxane, amino modified organopolysiloxane, silicone gel, acrylic silicone, trimethylsiloxysilicic acid, silicone RTV rubber, and the like.

The suitable blending amount of the oily ingredients are 0.5% by mass to 65% by mass, preferably 5% by mass to 60% by mass, and particularly preferably 10% by mass to 40% by mass, based on the total amount of the solid powder cosmetic.

### (Optional Ingredients)

The solid cosmetic according to the present embodiment may be manufactured by a publicly-known method depending on the intended dosage form and may appropriately contain optional ingredients as necessary, as long as the effect of the present embodiment is not impaired. Examples of the optional ingredients include thickeners, preservatives, surfactants, moisturizing agents, antioxidants, ultraviolet absorbers, sequestrants of metal ions, alcohols, sugars, amino acids, organic amines, polymer emulsions, pH adjusting agents, vitamins, antioxidants, anti-inflammatory agents, whitening agents, various extracts, activators, blood circulation promoting agents, anti-greasing agents, metal sequestrants, crude drug extracts, pharmaceutical agents, water, and the like. These optional ingredients may be contained in the powder ingredients and in the oily ingredients described above alone or in combination with two or more.

Examples of the thickeners include water-soluble polymers and clay minerals.

Examples of the water-soluble polymers include naturally occurring water-soluble polymers, semi-synthetic water-soluble polymers, and synthetic water-soluble polymers.

Examples of the naturally occurring water-soluble polymers include plant-based polymers (for example, gum arabic, tragacanth gum, galactan, guar gum, carob gum, gum karaya, carrageenan, pectin, agar, pyrus cydonia seed (pyrus cydonia), algae colloid (extract of Phaeophyta), starch (rice, corn, potato, wheat), glycyrrhizic acid, and the like); microbial polymers (for example, xanthan gum, dextran, succinoglycan, pullulan, and the like); animal-based polymers (for example, collagen, casein, albumin, dextrin, gelatin, and the like); and the like.

Examples of the semi-synthetic water-soluble polymers include starch-based polymers (for example, carboxymethyl starch, methyl hydroxypropyl starch, and the like); cellulose-based polymers (for example, methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powder, and the like); alginate-based polymers (for example, sodium alginate, propylene glycol alginate, and the like); and the like.

Examples of the synthetic water-soluble polymers include vinyl polymers (for example, polyvinyl alcohol, polyvinyl methyl ether, polyvinyl pyrrolidone, carboxyvinyl polymer, and the like); polyoxyethylene polymers (for example, polyoxyethylene-polyoxypropylene copolymers and the like of polyethylene glycol 20,000, 40,000, 60,0000); acrylic polymers (for example, sodium polyacrylate, polyethyl acrylate, polyacrylamide, and the like); polyethyleneimine; cationic polymers; and the like.

Examples of the clay minerals include aluminum magnesium silicate, bentonite, hectorite, A1Mg silicate (Veegum), laponite, silicate anhydride, and the like.

Examples of the preservatives include ethyl paraben, butyl paraben, chlorphenesin, phenoxyethanol, and the like.

Examples of the surfactants include anionic surfactants, cationic surfactants, amphoteric surfactants, nonionic surfactants, and the like.

Examples of the anionic surfactants include fatty acid soaps (for example, sodium laurate, sodium palmitate, and the like); higher alkyl sulfate salts (for example, sodium lauryl sulfate, potassium lauryl sulfate, and the like); alkyl ether sulfate salts (for example, POE-triethanolamine lauryl sulfate, sodium POE-lauryl sulfate, and the like); N-acyl sarcosinic acid (for example, sodium lauroyl sarcosine, and the like); higher fatty amide sulfonates (for example, sodium N-myristoyl-N-methyl taurate, sodium cocoyl methyl taurate, sodium lauryl methyl taurate, and the like); phosphate salts (sodium POE-oleylether phosphate, POE-stearylether phosphate, and the like); sodium sulfosuccinate (for example, di-2-ethylhexyl sulfosuccinate, monolauroyl monoethanolamide polyoxyethylene sulfosuccinate, sodium lauryl polypropylene glycol sulfosuccinate, and the like); alkyl benzene sulfonates (for example, sodium linear dodecyl benzene sulfonate, triethanolamine linear decyl benzene sulfonate, linear dodecyl benzene sulfonate, and the like); higher fatty acid ester sulfates (for example, sodium hardened glyceryl cocoate sulfate, and the like); N-acyl glutamates (for example, monosodium N-lauroyl glutamate, sodium N-stearoyl glutamate, monosodium N-myristoyl-L-glutamate, and the like); sulfated oils (for example, turkey red oil, and the like); POE-alkyl ether carboxylic acid; POE-alkylaryl ether carboxylate; α-olefin sulfonate; higher fatty acid ester sulfonate; secondary alcohol sulfate; higher fatty acid alkylolamide sulfate; sodium lauroyl monoethanolamide succinate; N-palmitoyl aspartate ditriethanolamine; sodium caseinate; and the like.

Examples of the cationic surfactants include alkyltrimethyl ammonium salts (for example, stearyltrimethyl ammonium chloride, lauryltrimethyl ammonium chloride, and the like); alkylpyridinium salts (for example, cetylpyridinium chloride, and the like); disteraryldimethyl ammonium dialkyldimethyl ammonium chloride, poly(N,N'-dimethyl-3,5-methylenepiperidinium) chloride; alkyl quaternary ammonium salts; alkyl dimethylbenzyl ammonium salts; alkyl isoquinolinium salts; dialkyl molifonium salts; POE-alkylamines; alkylamine salts; polyamine fatty acid derivatives; amyl alcohol fatty acid derivatives; benzalkonium chloride; benzetonium chloride; and the like.

Examples of the amphoteric surfactants include imidazoline amphoteric surfactants (for example, sodium 2-undecyl-N,N,N-(hydroxyethylcarboxymethyl)-2-imidazoline, 2-cocoyl-2-imidazolinium hydroxide-1-carboxyethyloxy disodium salt, and the like); betaine surfactants (for example, 2-heptadecyl-N-carboxymethyl-N-hydroxyethyl imidazolinium betaine, lauryl dimethylaminoacetate betaine, alkyl betaine, amidobetaine, sulfobetaine, and the like).

Examples of the lipophilic nonionic surfactants include sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglyceryl sorbitan penta-2-ethylhexanoate, diglyceryl sorbitan tetra-2-ethylhexanoate, and the like); glyceryl/polyglyceryl fatty acids (for example, cottonseed fatty acid monoglyceride, glyceryl monoerucate, glyceryl sesquioleate, glyceryl monostearate, glyceryl α,α'-pyroglutamate oleate, glyceryl monostearate malate, and the like); propylene glycol fatty acid esters (for example, propylene glycol monostearate, and the like); hardened castor oil derivatives; alkyl glyceryl ethers; and the like.

Examples of the moisturizing agents include polyethylene glycol, propylene glycol, glycerol, 1,3-butylene glycol, xylitol, sorbitol, maltitol, chondroitin sulfate, hyaluronic acid, mucoitin sulfate, caronic acid, atelocollagen, cholesteryl-12-hydroxy stearate, sodium lactate, bile salts, dl-pyrrolidone carboxylate, alkylene oxide derivatives, short chain soluble collagen, diglyceryl (EO)PO adducts, rosa roxburghii extract, melilot extract, and the like.

Examples of the antioxidants include tocopherols, dibutyl hydroxytoluene, butyl hydroxyanisole, gallic acid esters, and the like.

Examples of the ultraviolet absorbers include benzoic acid ultraviolet absorbers, anthranilic acid ultraviolet absorbers, salicylic acid ultraviolet absorbers, cinnamic acid ultraviolet absorbers, benzophenone ultraviolet absorbers, and the like.

Examples of the sequestrant of metal ions include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the alcohols include lower alcohols and polyhydric alcohols.

Examples of the lower alcohols include ethanol, propanol, isopropanol, isobutyl alcohol, t-butyl alcohol, and the like.

Examples of the polyhydric alcohols include divalent alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, and the like); trivalent alcohols (for example, glycerol, trimethylol propane, and the like); tetravalent alcohols (for example, pentaerythritol, such as 1,2,6-hexanetriol, and the like); pentahydric alcohols (for example, xylitol, and the like); hexavalent alcohols (for example, sorbitol, mannitol, and the like); polyhydric alcohol polymers (for example, diethylene glycol, dipropylene glycol, triethylene glycol, polypropylene glycol, tetraethylene glycol, diglycerol, polyethylene glycol, triglycerol, tetraglycerol, polyglycerol, and the like); divalent alcohol alkyl ethers (for example, ethylene glycol monomethyl ether, ethylene glycol monoethyl ether, ethylene glycol monobutyl ether, and the like); dihydric alcohol ethers (for example, diethylene glycol monomethyl ether, diethylene glycol monoethyl ether, and the like); dihydric alcohol ether esters (for example, ethylene glycol monomethyl ether acetate, ethylene glycol monoethyl ether acetate, and the like); glyceryl monoalkyl ether; sugar alcohols (for example, sorbitol, maltitol, maltotriose, mannitol, sucrose, erythritol, glucose, fructose, maltose, xylitol, and the like); and the like.

Examples of the sugars include trioses (for example, D-glyceryl aldehyde, dihydroxyacetone, and the like); tetroses (for example, D-erythrose, D-erythrulose, D-threose, erythritol, and the like); pentoses (for example, L-arabinose, D-xylose, L-lyxose, D-arabinose, D-ribose, D-ribulose, D-xylulose, L-xylulose, and the like); hexoses (for example, D-glucose, D-talose, D-psicose, D-galactose, D-fructose, L-galactose, L-mannose, D-tagatose, and the like); heptoses (for example, aldoheptose, heptose, and the like); octoses (for example, octulose, and the like); deoxy sugars (for example, 2-deoxy-D-ribose, 6-deoxy-L-galactose, 6-deoxy-L-mannose, and the like); amino sugars (for example, D-glucosamine, D-galactosamine, sialic acid, amino uronic acid, muramic acid, and the like); uronic acid (for example, D-glucuronic acid, D-mannuronic acid, L-gluronic acid, D-galacturonic acid, L-iduronic acid, and the like); oligosaccharides (for example, sucrose, guntianose, umbelliferose, lactose, planteose, isolychnoses, α,α-trehalose, raffinose, lychnoses, umbilicin, stachios stachyose verbascoses, and the like); polysaccharides (for example, cellulose, pyrus cydonia seed, chondroitin sulfate, starch, galactan, dermatan sulfate, glycogen, gum arabic, heparan sulfate, hyaluronic acid, tragacanth gum, keratan sulfate, chondroitin, xanthan gum, mucoytin sulfate, guar gum, dextran, keratosulfate, locust bean gum, succinoglucan, caronic acid, and the like); and the like.

Examples of the amino acids include neutral amino acids (for example, threonine, cysteine, and the like); basic amino acids (for example, hydroxylysine, and the like); and the like. Examples of the amino acid derivatives include sodium acyl sarcosinate (sodium lauroyl sarcosinate), acylglutamate, sodium acyl β-alanine, glutathione, pyrrolidone carboxylic acid, and the like.

Examples of the organic amines include monoethanolamine, diethanolamine, triethanolamine, morpholine, triisopropanolamine, 2-amino-2-methyl-1,3-propanediol, 2-amino-2-methyl-1-propanol, and the like.

Examples of the polymeric emulsions include acrylic resin emulsions, poly(ethyl acrylate) emulsions, acrylic resin solutions, polyacrylic alkyl ester emulsions, polyvinyl acetate resin emulsions, natural rubber latexes, and the like.

Examples of the pH adjusting agents include buffering agents using lactate and sodium lactate, citrate and sodium citrate, succinate and sodium succinate, and the like.

Examples of the vitamins include vitamins A, B1, B2, B6, C, E, and derivatives thereof, pantothenic acid, and derivatives thereof, biotin, and the like.

Examples of the antioxidant acids include phosphoric acid, citric acid, ascorbic acid, maleic acid, malonic acid, succinic acid, fumaric acid, kephalin, hexametaphosphate, phytic acid, ethylenediaminetetraacetic acid, and the like.

Examples of the anti-inflammatory agents include a glycyrrhizic acid derivative, a glycyrrhitinic acid derivative, a salicylic acid derivative, a hinokitiol, zinc oxide, allantoin, and the like.

Examples of the whitening agents include vitamin C, magnesium ascorbyl phosphate, ascorbic acid glucoside, arbutin, kojic acid, and the like.

Examples of the various extracts include extracts of phellodendron bark, coptis japonica, lithospermum root, chinese peony, swertia herb, birch, sage, loquat, carrot, aloe, mallow, iris, grape, coix seed, gourd, lily, saffron, cnidium officinale root, ginger root, hypericum erectum, ononis, garlic, capsicum, citrus unshiu peel, japanese angelica root, seaweed, and the like.

Examples of the activators include royal jelly, photosensitizers, cholesterol derivatives, and the like.

Examples of the blood circulation promoting agents include, for example, nonylic acid vanillylamide, nicotinic acid benzyl ester, nicotinic acid β-butoxyethyl ester, capsaicin, zingerone, cantharides tincture, ichthammol, tannic acid, α-borneol, tocopherol nicotinate, inositol hexanicotinate, cyclandelate, cinnarizine, tlazoline, acetylcholine, verapamil, cepharanthine, γ-oryzanol, and the like.

Examples of the anti-greasing agents include sulfur, thiantol, and the like.

Examples of the anti-inflammatory agents include tranexamic acid, thiotaurine, hypotaurine, and the like.

Examples of the metal sequestrants include disodium edetate, trisodium edetate, sodium citrate, sodium polyphosphate, sodium metaphosphate, gluconic acid, malic acid, and the like.

Examples of the crude drug extracts include caffeine, tannin, verapamil, tranexamic acid and its derivatives, licorice, chaenomeles fruit, pylolae herba, and the like.

Examples of the pharmaceutical agents include tocopherol acetate, glycyrrhitinic acid, glycyrrhizic acid, and its derivatives or salts, and the like.

### [Example]

Although the embodiments will be described in further detail with reference to the following examples, the embodiments are not limited by the examples.

### <Example 1>

### [Method of Manufacturing Solid Cosmetic]

Powder ingredients and oily ingredients described in the formula in Table 1 below were mixed using a Henschel mixer, and further ground using a pulverizer to obtain a homogeneous mixture. An equal amount of alcohol as a volatile dispersion medium was added to the mixture, and the mixture was mixed using a dispersion mixer to obtain a slurry. The obtained slurry was filled into an inner plate, preliminary pressing using a preliminary press head was performed, and then main pressing using a press head to obtain a molded body was performed. The molded body was dried at 55°C for 4 hours to obtain a solid cosmetic. The preliminary pressing and the main pressing were carried out according to the method described in the embodiments above. The blending amount of each ingredient used in the formula in Table 1 is in *%* by mass.

### <Evaluation of Solid Cosmetics>

The evaluation of the solid cosmetic was performed by measuring the radius of the corner of the decoration of the solid cosmetic, the maximum height difference in the irregularities of the decoration of the solid cosmetic, and the impact resistance.

### (Measurement of Radius of Corner of Decoration of Solid Cosmetic)

The radius of the corner of the decoration of the solid cosmetic was measured using a 3D scanner-type measuring instrument (Keyence).

### (Measurement of Maximum Height Difference in Irregularities of Decoration of Solid Cosmetic)

The maximum height difference of the irregularities of decoration of the solid cosmetic was measured using a 3D scanner-type measuring instrument (Keyence).

### (Evaluation of Impact Resistance)

The following criteria were used to evaluate whether the solid cosmetic was damaged when the solid cosmetic was dropped five times from the height of 30 cm.
GOOD: Not damaged
NOT GOOD: Damaged

**[Table 1]**

| | | | EXAMPLE 1 |
|---|---|---|---|
| FORMULA | POWDER INGREDIENTS | TALC | 12.0 |
| | | MICA | 5.0 |
| | | POLYETHYLENE POWDER | 5.0 |
| | | LIME SOAP | 4.0 |
| | | SYNTHETIC WAX POWDER | 2.0 |
| | | ZINC OXIDE | 1.0 |
| | | IRON OXIDE | 5.0 |
| | | IRON OXIDE COATED MICA TITANIUM | 50.0 |
| | OILY INGREDIENTS | VASELINE | 2.0 |
| | | DIISOSTEARYL MALATE | 6.0 |
| | | TRIETHYLHEXANOIN | 7.0 |
| | | DIMETHICONE | 1.0 |
| | TOTAL AMOUNT | | 100.0 |
| EVALUATION | RADIUS OF CORNER OF DECORATION [mm] | | 0.2 |
| | MAXIMUM HEIGHT DIFFERENCE OF IRREGULARITIES OF DECORATION [mm] | | 3.0 |
| | IMPACT RESISTANCE [TIMES] | | GOOD |

From Table 1, in Example 1, the radius of the corner of the decoration of the solid cosmetic was 0.2 mm, and the maximum height difference of the irregularities of the decoration was 3.0 mm. The impact resistance satisfied the standard required for use. Therefore, when the method of manufacturing a solid cosmetic according to the present embodiment is used, the solid cosmetic can have a decoration with a small corner radius and a large maximum height difference of irregularities, and have a high strength. Accordingly, a solid cosmetic having a sharply decorated surface can be formed with high productivity.

Although the embodiments have been described as above, the embodiments are presented by way of example and the invention is not limited by the embodiments. The embodiments may be implemented in various other forms, and various combinations, omissions, substitutions, modifications, and the like, may be made without departing from the spirit of the invention. These embodiments and modifications thereof are included in the scope and gist of the invention and fall within the scope of the claimed invention and equivalents thereof.

The present application claims priority to Japanese Patent Application No. 2020-080701, filed April 30, 2020, with the Japanese Patent Office. The contents of which are incorporated herein by reference in their entirety.

### [Description of the Reference Numeral]

- 1: Cosmetic mixture powder
- 2: Slurry
- 3: Molded body
- 4: Solid cosmetic
- 30: Inner plate
- 40: Pressing apparatus
- 41: Upper pressing apparatus
- 42: Lower pressing apparatus
- 43: Body
- 44: Press die (Press head)
- 44a: Press surface
- 44b: Through hole

## Claims

1. A method of manufacturing a solid cosmetic having a surface decorated with irregularities, the method comprising:
a pressing step of obtaining a molded body having a surface decorated with irregularities by press-molding a cosmetic composition containing a powder ingredient and an oily ingredient as a binder, using a press head including a press surface having irregularities corresponding to a surface shape of the solid cosmetic and a plurality of through holes penetrating through the press surface; and
a mold releasing step of, after the pressing step, separating the press surface and the molded body, wherein
the press head is a laminate made by laminating together a plurality of plates with holes,
the through holes are formed by connecting the holes of each of the plates, and
diameters of the through holes are 50 µm to 300 um.

2. The method according to claim 1, wherein in the pressing step, the press-molding is performed by bringing the press surface into direct contact with the cosmetic composition.

3. The method according to claim 1 or 2, wherein the pressing step includes:
a preliminary pressing step of temporarily pressing the cosmetic composition while the cosmetic composition is in a semi-dry state; and
a main pressing step of pressing a semi-dry product of the cosmetic composition obtained in the preliminary pressing step.

4. The method according to any one of claims 1 to 3, wherein the solid cosmetic includes, on the surface, an area where a radius of a corner of a decoration is 0.2 mm or less.

5. A press head for manufacturing a solid cosmetic having a surface decorated with irregularities, the press head comprising:
a press surface having irregularities corresponding to a surface shape of the solid cosmetic; and
a plurality of through holes penetrating through the press surface, wherein
the press head is a laminate made by laminating together a plurality of plates with holes,
the through holes are formed by connecting the holes of each of the plates, and
diameters of the through holes are 50 µm to 300 µm.

6. The press head according to claim 5, wherein an interval between adjacent through holes among the through holes on the press surface is from 200 um to 250 µm.
